# EUROPEAN PATENT SPECIFICATION

(11) **EP 2 754 434 B1**
(45) Date of publication and mention of the grant of the patent: **08.08.2018**
(21) Application number: 13000163.9
(22) Date of filing: 14.01.2013
(51) Int. Cl.: A61Q 11/00, A61K 8/92, A61K 8/9789

(54) **Composition for preventing dental erosion**
Zusammensetzung zum Verhindern von Zahnerosion
Composition destinée à prévenir l'érosion dentaire

(43) Date of publication of application: 16.07.2014
(73) Proprietor: Pandalis, Georgios, 49219 Glandorf (DE)
(72) Inventor: Pandalis, Georgios, 49219 Glandorf (DE)
(74) Representative: Grünecker Patent- und Rechtsanwälte PartG mbB

(56) References cited:
- WO-A1-02/069992
- WO-A1-2005/123101
- WO-A2-2006/027248
- US-A1- 2006 140 884
- KATO ET AL.: "Protective effect of green tea on dentin erosion and abrasion", JOURNAL OF APPLIED ORAL SCIENCE, vol. 17, no. 6, 2009, pages 560-564, XP002699979,

## Description

The present invention relates to a concentrate or extract of a plant for use in preventing dental erosion.

Besides caries, dental erosion and abrasions are the main cause for the loss of dental hard tissue. Dental erosion is defined as superficial dissolution of dental hard tissue without involvement of microorganisms.

Dental erosion is induced by intrinsic or extrinsic factors. Extrinsic factors are consumption of acidic beverages or foodstuffs, or may be work-related; the most common intrinsic factors are caused by gastric acid (gastroesophageal reflux, regurgitation, Anorexia, Bulimia nervosa).

In particular in most of the highly developed nations the extrinsic factors become more important due to increased consumption of fruits, fruit juices and acidic refreshment drinks. Furthermore, the use of highly abrasive toothpastes increases the removal of dental enamel.

As endogenous protective mechanism the enamel is protected by a biopolymer film called acquired pellicle, which is formed within seconds on the surface of the teeth after contact with saliva in the oral cavity. However, protection against acids is limited.

It has been found that the application of fluoride, e.g. fluoridated toothpaste, provides a protective effect against dental erosion.

Hannig et al., Caries Research 2012, 46, pages 496 to 506 describe the effect of oil pulling to prevent erosive damages. Furthermore, oil pulling with edible oils is part of body care in different culture groups. During oil pulling, edible oil is taken into the mouth, sucked, sipped and pulled between the teeth. It was assumed that oil pulling helps to prevent dental erosion by adding more hydrophobic properties to the tooth surface due to accumulation of lipids in the acquired pellicle. However, the study showed that oil pulling does not provide a protective effect.

Kato et al., Journal of Applied Oral Science 2009, 17, pages 560 to 564 studied the protective effect of green tea on dentin erosion. Dentin specimens from bovine incisors were placed in the oral cavity of the test person for 12 h to form the pellicle. The specimens were then immersed in a cup of Coke for 5 min at room temperature. The specimen were reinserted in the oral cavity and rinsed with 10 mL of green tea or water for 1 min. The results showed that the green tea reduces the dentin wear. However, rinsing with green tea can lead to tooth discoloration. Furthermore, green tea contains caffeine and is therefore not suitable for persons susceptible for caffeine.

WO-A-2005/123101 relates to the use of blackberry extracts to prevent and slow down periodontitis and the excessive degradation of periodontal connective tissue and damage to teeth caused by metalloproteinases.

WO-A-2006/027248 describes oral and dental care products containing substances which inhibit the formation of bacterial biofilms.

WO-A-02/069992 discloses extracts from a plant, which inhibit the activity of extracellular proteases.

US-A-2006/140884 relates to an oral care composition comprising one or more compounds of an extract of oregano and efficacious as antibacterial, anti-inflammatory, antiplaque, anti-oxidant and anti-halitosis composition.

The objective problem of the present invention is to maintain teeth healthy and to prevent dental erosion.

Said problem is solved by a composition for use in preventing dental erosion comprising a concentrate or extract of at least one plant selected from *Ribes nigrum,* wherein dental erosion is the superficial dissolution of dental hard tissue without involvement of microorganisms.

Preferred embodiments are set forth in subclaims 2 to 8.

### Description of the figures

Figure 1 shows the calcium and phosphate release of extracts of origanum and ribes at a pH of 2.0.
Figure 2 shows the calcium and phosphate release of extracts of origanum and ribes at a pH of 2.3
Figure 3 shows the calcium and phosphate release of extracts of origanum and ribes at a pH of 3.0

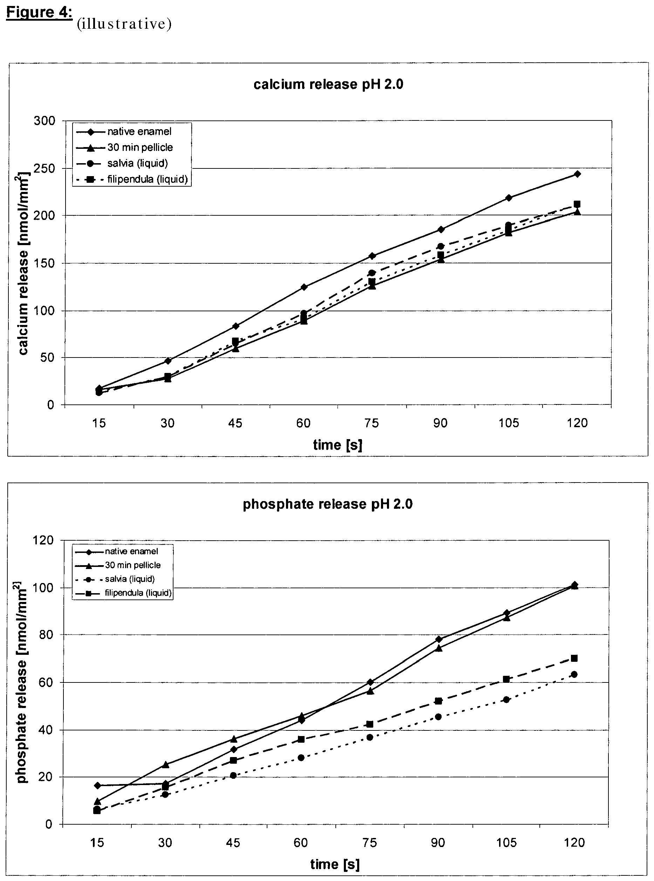

Figure 4 (illustrative) shows the calcium and phosphate release of extracts of salvia and filipendula at a pH of 2.0

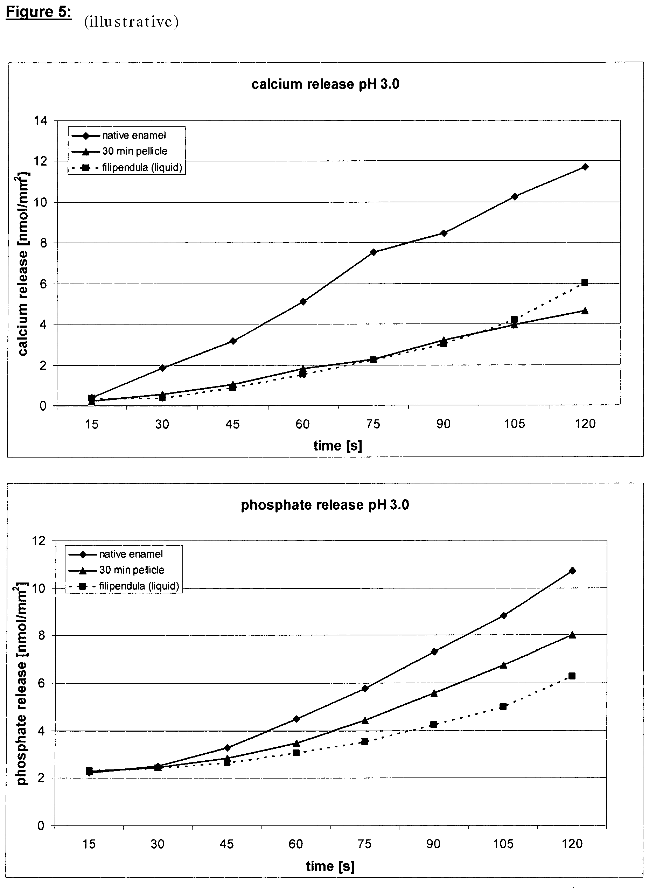

Figure 5 (illustrative) shows the calcium and phosphate release of extracts of filipendula at a pH of 3.0.

### Detailed description of the invention

The present invention relates to a composition for use in preventing dental erosion comprising a concentrate or extract of at least one plant selected from *Ribes nigrum,* wherein dental erosion is the superficial dissolution of dental hard tissue without involvement of microorganisms.

According to the invention, the term "preventing" is used for protection of teeth against dental erosion and thereby avoiding dental erosion.

According to the invention, the term "extract" is used representatively for all products that are obtained from an herbal subject by means of an extraction with a solvent, such as with maceration or percolation. The term "concentrate" is used representatively for all preparations that are obtained from an herbal subject by means of removal of water from the fresh plant. A dry plant product, such as a powder, is also included in the term "concentrate".

Generally, an extraction of the plant parts including leaves, twigs and fruits with a suitable solvent takes place. Suitable solvents are water, alcohols, such as methanol, ethanol or isopropyl alcohol, or chlorinated solvents, such as dichloromethane, as well as acetone, acetylacetone, ethylacetate, ammonia or glacial acetic acid, but also supercritical carbon dioxide. Mixtures of the solvents mentioned can also be used. In a preferred embodiment, in combination with any one of the embodiments listed above or below, water or a mixture of water with methanol or ethanol is used.

The extraction is normally carried out at temperatures of 25 °C to, where applicable, as high as the boiling point of the solvent used. Preferred is an extraction at 95 to 100 °C.

The extraction is normally carried out for 2 to 8 h. Preferably, the extraction is carried out for 3 to 6 h, more preferably for 4 to 5 h. Furthermore, fats, such as pork fat, waxes, such as beeswax, or oils, such as olive oil and almond oil, can be used for the extraction. Preferably, almond oil is used.

In order to achieve the highest possible yield, the plant material can be extracted a number of times. Preferably, the extraction is repeated 2 to 6 times, more preferably 3 times. In this case, it is also possible to use different solvents in the various extraction steps or an extraction with a solvent can be followed by an extraction with a fat, wax or oil, or vice versa.

As a result of the extraction, a liquid, semi-solid or solid raw product is obtained, which can be used in this form for producing a composition for the prophylaxis of dental erosion.

A maceration procedure is normally performed for five to nine days, preferably for seven days, at room temperature with a mixture of water and ethanol, by pouring the solvent mixture over the plant elements and letting this stand for the period of time mentioned.

According to the invention, a percolation of the plant parts is normally achieved by treating the parts with water at 95 to 100 °C for four to five hours by conducting the water through the plant parts.

The crude product obtained from an extraction with a solvent, such as a maceration or percolation, can also be concentrated and/or dried and/or further processed before use. The further processing can, for example, include cleaning steps known to the person skilled in the art, such as centrifugation, filtration and decanting, in order to remove suspended materials from the extract. Chromatography, such as column chromatography, gas chromatography or HPLC or steam distillation may also be used for purification. In a preferred embodiment the crude product is used without further purification steps.

An extract obtained in this way can subsequently be further processed into a dry extract. To produce the dry extract, the solvent can be withdrawn from the liquid raw extract, the concentrated extract or the cleaned extract by, for example, spray drying, freeze drying or vacuum drying.

In a preferred embodiment in combination with any one of the embodiments listed above or below, the concentrate or extract is from the aboveground parts of the plant, more preferably the aboveground parts are selected from the group consisting of leaves and twigs.

In the present invention the term "aboveground parts of a plant" refers to all parts which are aboveground, including leaves, twigs, blossoms, fruits and seeds. For preparing the concentrate or extract used according to the present invention, preferably the leaves and twigs are used. More preferably, the aboveground shoots of the plant that grow back in the same year are used. In general, all elements of the aboveground part of the plant, such as leaves, twigs, blossoms, fruits and seeds can be used. Preferably, the twigs are used with leaves and blossoms.

In the present invention, the term "twig" refers to a small shoot or branch having a diameter of 3 cm at most. Preferably, the diameter of the twigs is up to 1 cm.

In a preferred embodiment in combination with any one of the embodiments listed above or below, the composition of the present invention comprises a concentrate or extract of at least 2, 3, or 4 plants, more preferably of 2 or 3 plants, in particular of 2 plants.

*Ribes nigrum* contains flavonoids, terpenoids and essential oils in different concentrations. For example, the concentration may differ with respect to the leaves and fruits. Thus, in the following, the leaves and fruits are considered separately.

The scientific term *Ribes nigri folium* refers to the leaves of *Ribes nigrum,* whereas the term *Ribes nigri fructus* refers to the fruits of *Ribes nigrum.* Some of the components detected in *Ribes nigrum* are flavonols, such as quercetin and myricetin and their glycosides, as well as dimers or oligomers of proanthocyanidins. *Ribes nigri folium* comprises traces of essential oils, flavonolgylcoside and proanthocyanidine. *Ribes nigri fructus* contains anthocyanidins and flavonolglycosides, in particular isoquercitin, myricetin-D-glucopyranosid and rutosin. Further components of *Ribes nigri fructus* are fruit acids, such as citric acid, isocitric acid and malic acid, hydroxycinammic acid derivatives, Vitamin C and in the seeds γ-linolic acid (Hager's Handbuch der pharmazeutischen Praxis, Drogen P-Z, 5., vollständig neubearbeitete Auflage, Springer-Verlag, 1993, pages 466-474).

In another preferred embodiment in combination with one of the embodiments listed above or below, the composition of the present invention comprises a concentrate or extract *Ribes nigrum* and *Origanum vulgare,* or a concentrate or extract of *Ribes nigrum* and *Filipendula ulmaria.* More preferably, the composition of the present invention comprises a concentrate or extract of *Ribes nigrum* and *Origanum vulgare.*

Origanum is a genus belonging to the family of *Lamiaceae* covering about 20 species of aromatic herbs, native to the Mediterranean Basin east to eastern Asia. The genus of origanum includes oregano and marjoram.

Of particular interest in the present invention is *Origanum vulgare* (oregano)

*Herba Origani vulgaris,* the herb of oregano, contains essential oils with carvacrol as main component, and further γ-terpinen, p-cymene, α-pinen, myrcen, α-terpinen and thymol. It further contains flavons and flavonglycosides, such as naringin, luteolin, apigenin, leptosidin, peonidin, catechin; phenol carboxylic acids and derivatives thereof, such as rosmarinic acid, cinnamic acid, caffeic acid, p-hydroxybenzoic acid, vanillic acid, syringic acid, protocatechuic acid, 4-(3,4-dihydroxybenzoyloxymethyl)-phenyl-β-D-glucopyranoside and 2-caffeoyloxy-3-[2-(4-hydroxybenzyl)-4,5-dihydroxy]phenylpropionic acid. The oil of oregano, *Origani aetheroleum,* is obtained by steam distillation of the fresh or dried herb and contains terpenes, such as carvacrol, γ-terpinen, p-cymene, α-pinen, myrcen, α-terpinen and thymol. Further ingredients are camphen, β-pinen, limonen, 1,8-cineol, 1-octen-3-ol, campher, linalool, terpinen-4-ol, β-carophyllen, α-terpineol, borneol, and bornyl acetate (Hager's Handbuch der pharmazeutischen Praxis, Drogen E-O, 5., vollständig neubearbeitete Auflage, Springer-Verlag, 1993, pages 959-966).

Filipendula is a genus belonging to the family of Rosaceae covering 12 species of perennial herbaceous flowering plants native to the temperate regions of the Northern Hemisphere.

Of particular interest in the present invention is *Filipendula ulmaria.*

The flowers of *Filipendula ulmaria, Spiraeae flos,* contain essential oils, salicyl aldehyde, salicyl acid methyl ester; flavonols such as spiraeosid and the corresponding kaempferol derivative, quercetin and its derivatives (hyperoside, rutin, quercetin-3-glucuronide and quercetin-3-arabinoside); phenol glycosides such as monotropitin, spiraein, isosalicin; tannins, such as gallotannin and ellagitannin. The herb of *Filipendula ulmaria, Spiraeae herba,* contains mainly the same ingredients, but in different ratios. The leaves do not contain spiraein, or contain only traces of this compound (Hager's Handbuch der pharmazeutischen Praxis, Drogen E-O, 5., vollstandig neubearbeitete Auflage, Springer-Verlag, 1993, pages 147-155).

In a further preferred embodiment of the invention, in combination with any one of the embodiments listed above or below, the composition according to the invention comprises a concentrate or an extract of two different plants in a ratio of 1 : 10 to 10 : 1, preferably 1 : 5 to 5 : 1, and more preferably 1 : 2 to 2 : 1 and in particular 1 : 1. More preferably, the composition comprises a concentrate or extract of *Ribes nigrum* and *Origanum vulgare,* or a concentrate or extract of *Ribes nigrum* and *Filipendula ulmaria* in a ratio of 1 : 10 to 10 : 1, preferably 1 : 5 to 5 : 1, and more preferably 1 : 2 to 2 : 1 and in particular 1 : 1. In particular, the composition according to the invention comprises a concentrate or an extract of *Ribes nigrum* and *Origanum vulgare* in a ratio of 1 : 1.

In another preferred embodiment in combination with one of the embodiments listed above or below, the composition of the present invention is in liquid, dry or semi-solid form.

The composition can be applied in each of the application forms familiar to the person skilled in the art for both medical and non-medical use. In a preferred embodiment in combination with one of the embodiments listed above or below, the composition is applied in solid form as a tablet, such as a coated tablet, effervescent tablet, chewing tablet, chewing gum, capsule, powder, granulate, sugar-coated tablet, lozenge, or pill. In an alternative preferred embodiment in combination with one of the embodiments listed above or below, the composition is applied in liquid form as mouthwash, sirup, drops, tincture, emulsion, or gargling solution. In another preferred embodiment in combination with one of the embodiments listed above or below, the composition is applied in a semi-solid form as ointment, cream, gel, or paste, e.g. toothpaste. In a further preferred embodiment in combination with one of the embodiments listed above or below, the composition is applied as a spray, such as a mouth spray. In particular, the composition is applied as a tablet, sirup, mouthwash or toothpaste.

In a further preferred embodiment in combination with one of the embodiments listed above or below, the extract of at least one plant is an aqueous extract, an alcoholic extract or an oily extract.

In galenic and other application forms, the composition can be processed with the customary galenic aids, such as tablet bonders, filling agents, preservative agents, tablet-opening agents, flow regulation agents, softening agents, wetting agents, dispersing agents, emulsifying agents, solvents, retarding agents, anti-oxidative agents, consistency regulators, penetration improvers and/or propellant gases. Further elements, such as vitamins and minerals, can be added to the composition according to the invention. Of particular interest is a composition according to the invention further comprising fluoride. The fluoride can be added directly to the composition in the form of a salt, such as sodium fluoride, or in the form of an extract or concentrate of a fluoride-containing plant, such as nasturtium, water cress and black tea.

The concentration of the composition in the application form varies, depending on the type of application. As a rule, the quantity of the composition amounts to between 0.5 and 1,000 mg per dosing unit for solid application forms. Preferably the quantity of the composition amounts to between 1 and 500 mg per unit. In liquid application forms, the composition can be in a concentration of 0.1 µg/mL to 500 mg/ml, preferably from 1 µg/mL to 200 mg/mL, more preferably from 1 to 100 mg/mL, most preferably 20 to 50 mg/mL, in particular 25 to 35 mg/mL. In the case of semi-solid application forms, the content of the composition amounts to 1 to 90% by weight, preferably 5 to 75 % by weight, based on the total weight of the application form.

The following examples further explain the present invention.

### Examples

### General procedure for the production of liquid and dry extracts:

The collected plant material is visually checked, and non-original, damaged or eaten away parts are removed.

The purified material is spread on a table in a green house and covered with paper for drying. The plant parts are turned around on a daily basis and visually checked for non-original and damaged parts, which are removed. The residual moisture of the plant material is determined on regular basis. The material is good for further processing when the residual water content is at a maximum of 10% by weight. Alternatively, the purified material is dried with a belt dryer according to standard procedures known to the person skilled in the art.

The plant material is coarsely cut and transferred to a beaker; Cold, distilled water is added (10 to 30-times the quantity of the plant material). The resulting mixture is heated on a hotplate while being stirred until it starts boiling. Simmering is continued for 1 h.

The hot liquid is strained, and the residual plant material is squeezed out. The resulting aqueous extract is filled directly in a bottle.

For the production of the dry extract, the liquid extract is filled in metal bowls and concentrated at 80 °C in a drying oven until the solvent has completely evaporated. The residue is scraped out of the metal bowl and weighed. Alternatively, the liquid extract is freeze-dried according to standard procedures known to the person skilled in the art.

### Preparation of an extract of Origanum vulgare

All above-ground parts of *Origanum vulgare* are used for the extraction.

Following the general procedure, 100 g of coarsely cut *Origanum vulgare* is heated in 1000 mL of distilled water. The resulting mixture is heated on a hotplate while being stirred until it starts boiling. Simmering is continued for 1 h. The resulting aqueous extract is filled directly in a bottle.

To obtain a dry extract, the liquid extract is dried according to the general procedure. The content of dry substance is at least 92 % by weight, based on the total weight of the dry extract.

### Preparation of an extract of Ribes nigrum

The leaves of *Ribes nigrum* are used for the extraction.

Following the general procedure, 33.3 g of coarsely cut *Ribes nigrum* is heated in 1000 mL of distilled water. The resulting mixture is heated on a hotplate while being stirred until it starts boiling. Simmering is continued for 1 h. The resulting aqueous extract is filled directly in a bottle.

To obtain a dry extract, the liquid extract is dried according to the general procedure. The content of dry substance is at least 92 % by weight, based on the total weight of the dry extract.

### Preparation of an extract of Salvia officinalis

All above-ground parts of *Salvia officinalis* are used for the extraction.

Following the general procedure, 33.3 g of coarsely cut *Salvia officinalis* is heated in 1000 mL of distilled water. The resulting mixture is heated on a hotplate while being stirred until it starts boiling. Simmering is continued for 1 h. The resulting aqueous extract is filled directly in a bottle.

### Preparation of an extract of Filipendula ulmaria

All above-ground parts of *Filipendula ulmaria* are used for the extraction.

Following the general procedure, 33.3 g of coarsely cut *Filipendula ulmaria* is heated in 1000 mL of distilled water. The resulting mixture is heated on a hotplate while being stirred until it starts boiling. Simmering is continued for 1 h. The resulting aqueous extract is filled directly in a bottle.

### Preparation of a liquid extract of Origanum vulgare and Ribes nigrum

All above-ground parts of *Origanum vulgare* and the leaves of *Ribes nigrum* are used for the extraction.

Following the general procedure, 70 g of coarsely cut *Origanum vulgare* and 30 g of coarsely cut *Ribes nigrum* leaves are boiled in 1000 mL of distilled water for 1 h. The resulting aqueous extract is filtered and boiled again for 5 min. The resulting extract is filled directly in a bottle. The concentration of the liquid extract is 30.5 mg/mL.

### Preparation of a dry extract of Origanum vulgare and Ribes nigrum

### Preparation of a dry extract of Origanum vulgare

All above-ground parts of *Origanum vulgare* are used for the extraction.

Following the general procedure, 150 g of coarsely cut *Origanum vulgare* is heated in 1500 mL of distilled water for 1 h. The resulting aqueous extract is dried according to standard procedures known to the person skilled in the art. The content of dry substance is at least 92 % by weight, based on the total weight of the dry extract.

### Preparation of a dry extract of Ribes nigrum

The leaves of *Ribes nigrum* are used for the extraction.

Following the general procedure, 26.7 g of coarsely cut *Ribes nigrum* leaves are heated in 800 mL of distilled water for 1 h. The resulting aqueous extract is dried according to standard procedures known to the person skilled in the art. The content of dry substance is at least 92 % by weight, based on the total weight of the dry extract.

### Preparation of a dry extract of Origanum vulaare and Ribes nigrum

The dry extract of *Origanum vulgare* is mixed thoroughly with the dry extract of *Ribes nigrum* in a weight ratio of 1 : 1.

### Determination of Calcium and Phosphate Release

### Subjects

A number of 3 healthy volunteers participated in the study. Visual oral examination was carried out by an experienced dentist. The subjects showed no signs of gingivitis or caries; they had a physiological salivary flow rate as determined qualitatively during oral examination.

### Specimen preparation

Enamel slabs with a diameter of 5 mm were prepared from bovine incisor teeth (2-year-old cattle). According to the size of the teeth, between 2 and 3 samples were gained from each bovine incisor. Specimens were etched at all sites except for the outer enamel surface for 15 s with 37% phosphoric acid gel (Etching gel, DMG, Hamburg, Germany) and treated for 30 s with Optibond Primer (Kerr, Karlsruhe, Germany). Optibond Adhesive was applied afterwards and light-cured for 30 s. Application of the adhesive was performed 3 times. Subsequently, the unsealed enamel surfaces were ground flat and polished (grit 4000). In a standardized grinding procedure, approximately 200 µm of the enamel was removed. Specimens with structural alterations of the enamel were excluded from the study. The smear layer on the slabs was removed with a steam jet and by ultrasound with NaOCI for 3 min. Then the slabs were washed twice in distilled water for 5 min also by ultrasound. Afterwards, the samples were disinfected in ethanol (70%) for another 10 min (ultrasound), washed in distilled water and stored in distilled water for 24 h before exposure in the oral cavity.

The above extracts were tested to evaluate the effect on the acquired pellicle and on erosive mineral loss.

### In situ Pellicle Formation and in vitro Erosion

For in situ pellicle formation, individual upper jaw splints were vacuum-formed from 1.5-mm-thick methacrylate foil for each volunteer. Cavities were prepared in the buccal aspects of the splints (in the region of 16, 15, 14, and the region of 24, 25, 26, respectively). A number of 6 slabs were fixed on each splint with polyvinylsiloxane impression material (President Light Body, Coltène, Switzerland) in such a way, that only the specimen's surfaces were exposed to the oral environment. The splints were carried intraorally for 1 min to allow initial pellicle formation on the specimen surfaces. Afterwards, in case of a liquid extract, the subjects rinsed thoroughly with the extracts for 10 min. In case of a dry extract in the form of a tablet, the tablet was allowed to dissolve for 10 min. The splints remained in the oral cavity for further 19 min; this regime gave a total intraoral exposure time of 30 min. After intraoral exposure, the slabs were quickly removed from the splints and rinsed thoroughly with running water for 5 s. In vitro erosion was performed by incubating each sample in 1,000 µL hydrochloric acid (pH 2, 2.3, 3) to provide an excess of acid in order to maintain constant pH during the incubation period. Before exposure to HCI, the enamel slabs were embedded in silicone impression material at the bottom of a 2-ml Eppendorf cup. The acid was moved continuously by pumping with a 100 µL pipette (1 lift/s). Every 15 s, 100 µL of the acid was removed for photometric analysis and replaced by 100 µL of fresh acid. For control purposes, enamel specimens coated with a 30-min salivary pellicle (without rinsing) and specimens without pellicle were incubated in HCl.

### Photometric Determination of Calcium and Phosphate Release

Mineral dissolution caused during incubation in HCI was determined by measuring photometrically calcium and phosphate release into the solution in double assays using the arsenazo III method (Fluitest, Ca-A-II, Analyticon, Lichtenfels, Germany) and the malachite green assay, as described previously. Arsenazo III reacts with calcium in an acidic solution to form a blue purple complex. The resulting intensity is proportional to the calcium concentration. Absorption can be determined at λ = 650 nm according to standard curves. The reagent for determination of calcium was composed of 100 mM imidazole buffer (pH 6.5) and 0.12 mM arsenazo III. A volume of 10 µL from the sample was pipetted to 100 µL arsenazo reagent. Two samples were measured for each specimen, and the average absorption was calculated. Malachite green reacts with phosphate to form a colored complex which can be determined photometrically at λ = 650 nm. For the test reagent 0.045 mg of malachite green dissolved in 100 mL distilled water was mixed with 12.69 g of ammonium molybdate dissolved in 300 mL HCI (4 M). The reagent was stirred for 30 min afterwards and filtered (pore size 0.22 µm). A volume of 10 mL from the sample was pipetted to 200 µL malachite reagent; the absorption was measured after 15 min. Two samples were measured for each specimen, and the average absorption was calculated. Calcium and phosphate release were calculated based on the mean photometric absorption values for the specimens and their surface areas (based on 5 mm diameter).

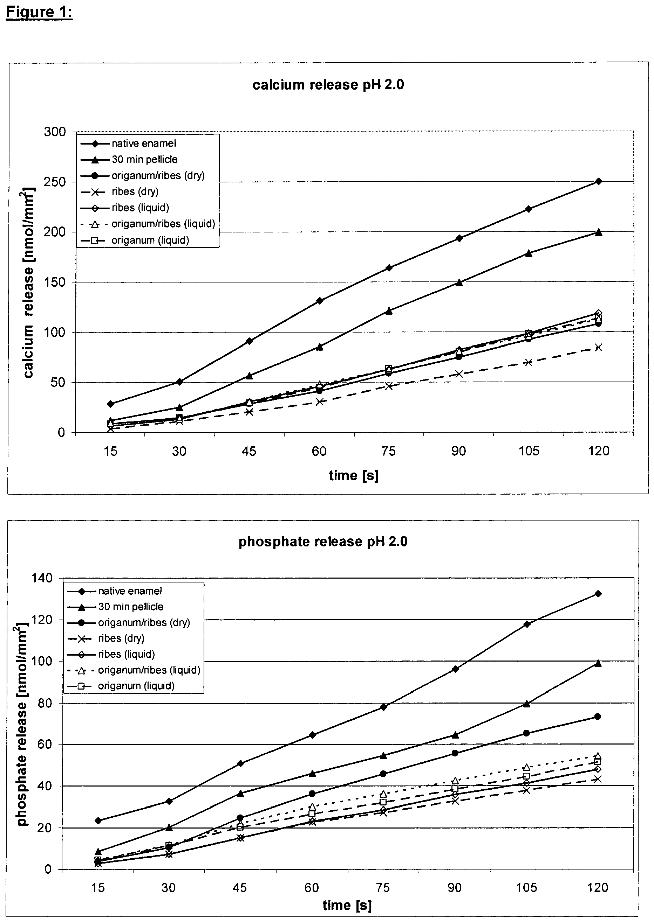

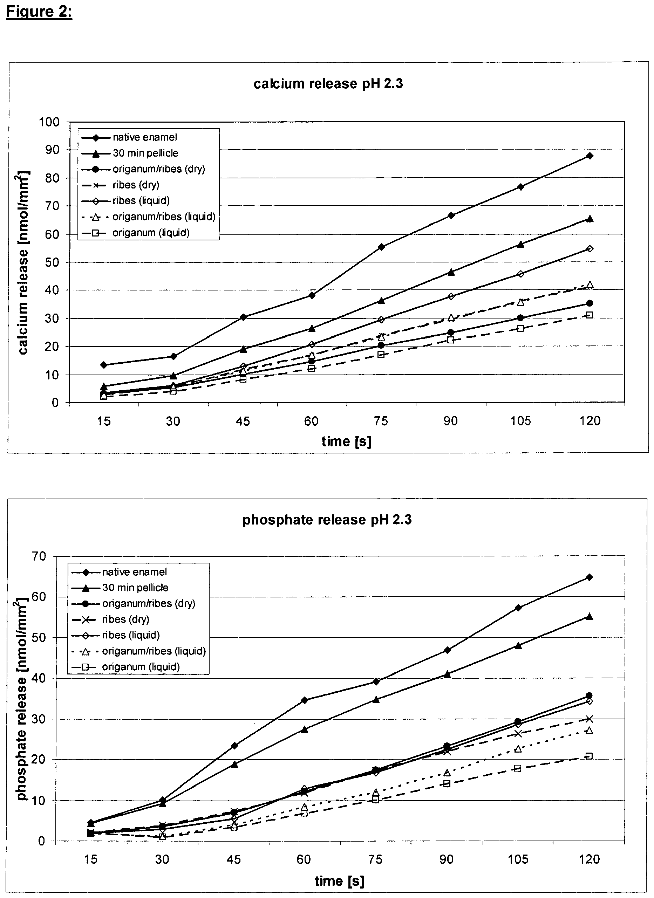

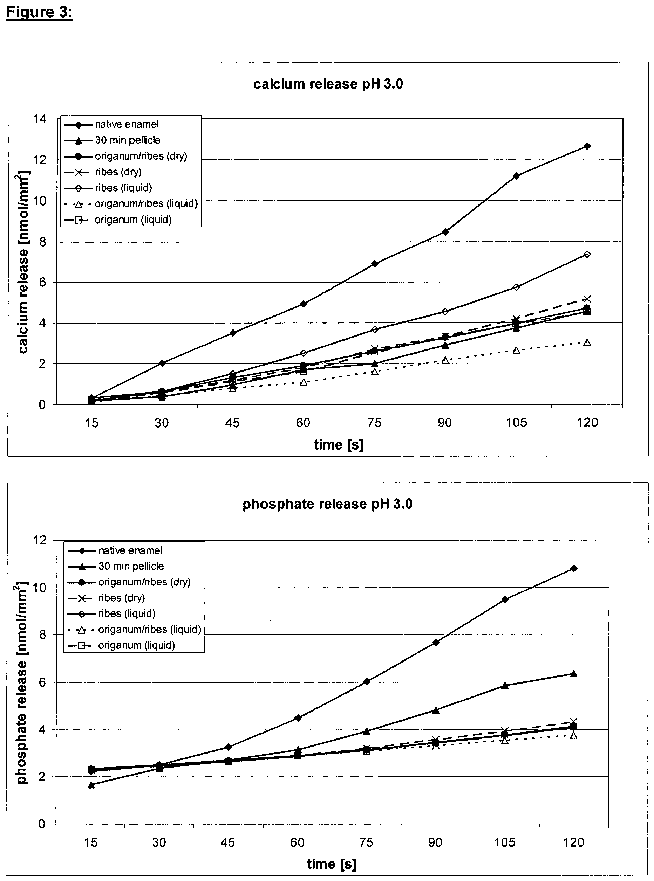

As can be seen from Figures 1 to 3, when treating the enamel slabs with the extracts of the present invention, less calcium and phosphate is released. Therefore, the extracts in accordance with the present invention are able to prevent dental erosion.

## Claims

1. Composition for use in preventing dental erosion, comprising a concentrate or extract of at least one plant selected from *Ribes nigrum,* wherein dental erosion is the superficial dissolution of dental hard tissue without involvement of microorganism.

2. The composition for use according to claim 1 wherein the concentrate or extract is from the aboveground parts of the plant.

3. The composition for use according to claim 1 or 2, comprising a concentrate or extract of at least 2, 3, or 4 plants.

4. The composition for use according to any one of claims 1 to 3, comprising a concentrate or extract of *Ribes nigrum* and *Origanum vulgare.*

5. The composition for use according to any one of claims 1 to 3, comprising a concentrate or extract of *Ribes nigrum* and *Filipendula ulmaria.*

6. The composition for use according to any one of claims 1 to 5, wherein the composition is in liquid, dry or semi-solid form.

7. The composition for use according to any one of claims 1 to 6, wherein the extract is an aqueous extract, an alcoholic extract or an oily extract.

8. The composition for use according to any one of claims 1 to 7, wherein the composition is in the form of a tablet, mouthwash, sirup, drops, tincture, emulsion, gargling solution, ointment, cream, gel, paste or mouth spray.

## Patentansprüche

1. Zusammensetzung zur Verwendung in der Vorbeugung von Zahnerosion, umfassend ein Konzentrat oder einen Extrakt von mindestens einer Pflanze ausgewählt aus *Ribes nigrum,* wobei Zahnerosion die oberflächliche Auflösung von Zahnhartgewebe ohne Beteiligung von Mikroorganismen ist.

2. Zusammensetzung zur Verwendung nach Anspruch 1, wobei das Konzentrat oder der Extrakt von den oberirdischen Teilen der Pflanze ist.

3. Zusammensetzung zur Verwendung nach Anspruch 1 oder 2, umfassend ein Konzentrat oder Extrakt von mindestens 2, 3 oder 4 Pflanzen.

4. Zusammensetzung zur Verwendung nach einem der Ansprüche 1 bis 3, umfassend ein Konzentrat oder Extrakt von *Ribes nigrum* und *Origanum vulgare.*

5. Zusammensetzung zur Verwendung nach einem der Ansprüche 1 bis 3, umfassend ein Konzentrat oder Extrakt von *Ribes nigrum* und *Filipendula ulmaria.*

6. Zusammensetzung zur Verwendung nach einem der Ansprüche 1 bis 5, wobei die Zusammensetzung in flüssiger, trockener oder halbfester Form vorliegt.

7. Zusammensetzung zur Verwendung nach einem der Ansprüche 1 bis 6, wobei der Extrakt ein wässriger Extrakt, ein alkoholischer Extrakt oder ein öliger Extrakt ist.

8. Zusammensetzung zur Verwendung nach einem der Ansprüche 1 bis 7, wobei die Zusammensetzung in der Form einer Tablette, Mundwasser, Sirup, Drops, Tinktur, Emulsion, Gurgellösung, Salbe, Creme, Gel, Paste oder Mundspray vorliegt.

## Revendications

1. Composition destinée à l'utilisation dans la prévention de l'érosion dentaire, comprenant un concentré ou un extrait d'au moins une plante sélectionnée parmi *Ribes nigrum,* l'érosion dentaire étant la dissolution superficielle du tissu dentaire dur sans implication de micro-organisme.

2. Composition destinée à l'utilisation selon la revendication 1, le concentré ou l'extrait provenant des parties aériennes de la plante.

3. Composition destinée à l'utilisation selon la revendication 1 ou 2, comprenant un concentré ou un extrait d'au moins 2, 3, ou 4 plantes.

4. Composition destinée à l'utilisation selon l'une quelconque des revendications 1 à 3, comprenant un concentré ou un extrait de *Ribes nigrum* et *d'Origanum vulgare.*

5. Composition destinée à l'utilisation selon l'une quelconque des revendications 1 à 3, comprenant un concentré ou un extrait de *Ribes nigrum* et de *Filipendula ulmaria.*

6. Composition destinée à l'utilisation selon l'une quelconque des revendications 1 à 5, la composition se présentant sous une forme liquide, sèche ou semi-solide.

7. Composition destinée à l'utilisation selon l'une quelconque des revendications 1 à 6, l'extrait étant un extrait aqueux, un extrait alcoolique ou un extrait huileux.

8. Composition destinée à l'utilisation selon l'une quelconque des revendications 1 à 7, la composition se présentant sous la forme d'un comprimé, d'un lavage buccal, d'un sirop, de gouttes, d'une teinture, d'une émulsion, d'une solution pour gargarisme, d'un onguent, d'une crème, d'un gel, d'une pâte ou d'une pulvérisation buccale.
